# EUROPEAN PATENT SPECIFICATION

(11) **EP 2 231 245 B1**
(45) Date of publication and mention of the grant of the patent: **29.10.2014**
(21) Application number: 07861143.1
(22) Date of filing: 20.12.2007
(51) Int. Cl.: A61M 16/00

(54) **A computer program product, a control unit for a ventilator, and a ventilator**
Computerprogramm-produkt, kontrollgerät für ein beatmungsgerät, und beatmungsgerät
Produit de programme informatique, unité de commande pour un respirateur, et respirateur

(43) Date of publication of application: 29.09.2010
(73) Proprietor: Maquet Critical Care AB, 171 06 Solna (SE)
(72) Inventor: LAKSOV, Joakim, S-182 56 Danderyd (SE); LAGERBORG, Johan, S-144 42 Rönninge (SE)
(74) Representative: Norberg, Charlotte
(86) International application number: PCT/SE2007/051048
(87) International publication number: WO 2009/082295

(56) References cited:
- WO-A-00/00245
- WO-A-99/43374
- US-A- 4 440 176
- US-B1- 6 411 843

## Description

### Technical Field

The present invention relates to a ventilator for use in support mode and a method for ventilating a patient in support mode.

### Background and Related Art

A ventilator for providing breathing support to a patient can work in different modes, depending, i.a. on the patient's condition. If the patient is showing some breathing activity a support mode is often suitable, in which the ventilator provides extra breathing support in phase with the patient's own breathing activity. In this case the patient's own breathing activity must be monitored in an appropriate way in order to synchronize the breathing support provided by the ventilator with the patient's own breathing so that an inspiration phase is started by the ventilator when the patient starts inhaling. Typically a pneumatic trigger condition based on pressure and/or flow in the ventilator is set.

In some cases it is difficult to synchronize the ventilation correctly with the patient's breathing efforts. For example, if there is a leakage, it will be difficult to set a suitable pneumatic trigger level. Finding a suitable level may require a lot of trial and error. In particular, when ventilating children leakages generally occur, since in that case a cuff is typically not used around the tube.

Imperfect synchronization between the ventilator's and the patient's breathing cycles can lead to increased work for the patient. If the trigger is too insensitive an entire breath may be skipped. If the trigger is too sensitive an inspiration may be triggered in the ventilator when the patient is not ready to inhale.

Various methods improving the trigger by means of bioelectric signals are known from WO 99/43374 and WO 00/00245.

### Object of the Invention

It is an object of the invention to improve the trigger conditions when ventilating a patient in support mode

### Summary of the Invention

This object is achieved according to the present invention by a computer program product comprising a computer readable medium having stored thereon computer readable code means for controlling a ventilator providing breathing support in a support mode to a patient, said computer program product comprising computer readable instructions which, when run in a control unit controlling a ventilator will cause the control unit to perform the following steps:
- obtaining a measurement value of a bioelectric signal representative of the patient's own breathing function;
- determining, based on the bioelectric signal, at least one point in time in which the patient starts inhalation,
- obtaining a measurement value related to a pressure and/or flow to be used for triggering an inspiration phase in the ventilator during said at least one point in time,
- determining a trigger condition for the inspiration phase on the basis of the measurement value,
- using the trigger condition to for initiating inspiration when ventilating the patient in support mode.

According to the invention, therefore, Edi signal is used to determine the point in time when the patient starts inhaling, and the triggering conditions for an inspiration phase in the ventilator may be adjusted based on measurements of pressure and/or flow performed in the ventilator at this point in time. The invention therefore facilitates ventilation in pneumatic mode that is adapted to the patient's own breathing cycle.

As will be understood by the skilled person, the method is performed by a computer program, preferably located in the control unit of the ventilator for controlling the ventilator. Hence, the invention also relates to a control unit for a ventilator comprising a computer program product as defined above and a ventilator comprising such a control unit.

Preferably, the control unit will be caused to adjust the trigger condition by an amount determined on the basis of the measurement value.

In one embodiment the code means is arranged to cause the control unit to determine the trigger condition on the basis of several measurement values, each obtained at a point in time when the patient starts inhalation, in different breaths. This will provide a more accurate value for the trigger condition.

In a preferred embodiment, the code means comprises computer readable instructions which, when run in a control unit controlling a ventilator will cause the control unit, after determining the start of inhalation and before measuring the pressure, to determine whether an inspiration phase in the ventilator was triggered before the start of inhalation and, if so, slightly delaying the ventilator's inspiration phase until the patient's own breathing attempt can be detected.

In one embodiment the code means is arranged to cause the control unit to adjust the trigger condition incrementally in such a way as to reduce the time difference between start of the ventilator's inspiration phase and the start of the patient's inspiration. The trigger condition may be adjusted, for example, in fixed increments or in increments determined on the basis of the difference between the measurement value and the trigger condition.

### Brief Description of the Drawings

The invention will be described in more detail in the following, by way of example and with reference to the appended drawings in which:
Figure 1 illustrates a ventilator providing breathing support to a patient.
Figure 2 illustrates a situation where the trigger condition is too sensitive.
Figure 3 illustrates a situation where the trigger condition is too insensitive.
Figure 4 is a flow chart of an embodiment of the inventive method.
Figure 5 is a more detailed flow chart of one of the steps of Figure 4.

### Detailed Description of Embodiments

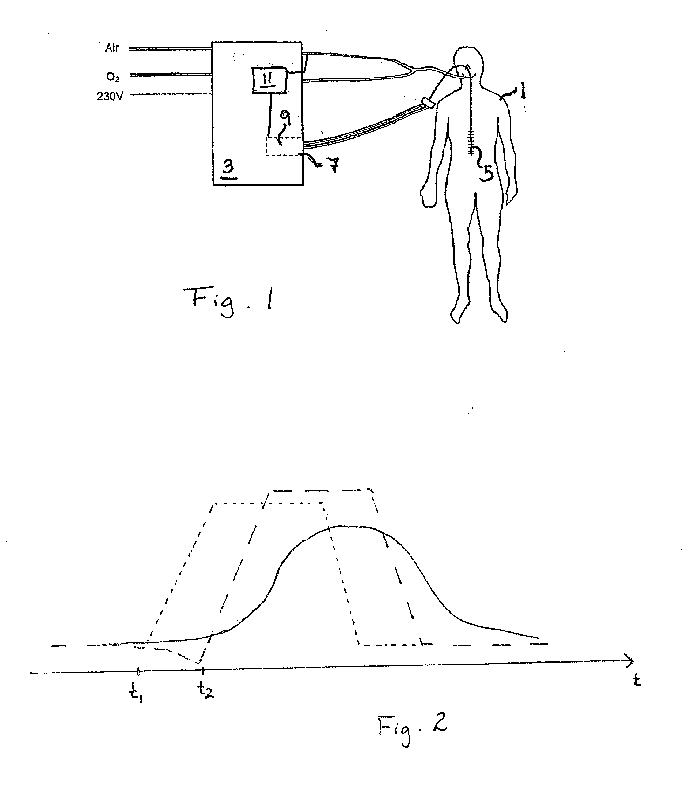

Figure 1 is a schematic overview of a patient 1 connected to a ventilator 3. The ventilator is arranged to work in support mode but can also be arranged to work in a controlled mode. To capture the Edi signal, the patient 1 has an oesophageal catheter 5 inserted in order to record a myoelectric signal from the diaphragm. This myoelectric signal (EMG signal) is fed to a control input 7 of the ventilator 3 and processed in a control unit 9 in the ventilator to produce the overall signal, called an Edi signal. According to the invention, a bioelectric signal related to breathing, such as the Edi signal is used to adjust the pneumatic triggering criteria of the ventilator.

Typically the ventilator comprises registration means 11 for monitoring the pressure and/or flow of breathing gas in the breathing circuit 1. The control unit 9 comprises processor means and at least one computer program arranged to compare the patient's own breathing activity to the breathing support provided by the ventilator and to adjust, if necessary, the triggering of the inspiration to synchronize it better with the patient's own breathing. This will be discussed in more detail below.

In order to assist the patient's breathing in such a way that the patient's own attempts to inhale air are supported by an additional flow of breathing gas from the ventilator, the flow and/or pressure in the ventilator is measured. The pressure and/or flow sensors in the ventilator are used together with the trigger settings in order to detect the patient's attempt to inhale and initiate the inspiration phase. This is referred to as pneumatic triggering. Alternatively, an increased flow in the direction towards the patient is measured. Ideally the start of this inspiration phase should be perfectly synchronized with the start of the patient's own inhalation. To achieve this, the threshold value for the flow and/or pressure must be set correctly so that the flow and/or pressure measured in the ventilator will pass the threshold at exactly the same time as the patient starts inhaling. This is not always the case, as will be discussed in the following.

Figure 2 illustrates a situation in which the pneumatic trigger function is based on pressure measurements and is too sensitive, causing the inspiration to be triggered too early in the breathing cycle compared to the patient's own breathing. Three curves are shown varying along a time axis denoted t. The solid curve represents the Edi signal recorded in the patient, that is, it reflects the patient's own breathing activity. The positive flank represents an inhalation by the patient. The dashed curve is an ideal ventilator cycle, starting inspiration (positive flank) when the patient starts inhaling. The dotted curve is an example of the breathing support that will result if the trigger condition is too sensitive. In this case, the triggering should be made to start later to be in phase with the patient's own breathing.

Too early triggering, as illustrated in Figure 2, may be caused, for example, by a leakage in the breathing circuit or if there is water in the tubes. It may also be due to oscillations caused by variations in the patient's thorax, caused by heart activity. In the case shown in Figure 2, the pressure will drop below the trigger condition at a first point in time t1, which occurs before the patient actually starts inhaling, at a second point in time t2. Hence, the ventilator will start inspiration support before the patient is ready to inhale. In this case, therefore, the pneumatic triggering should be delayed to be in phase with the patient's own breathing.

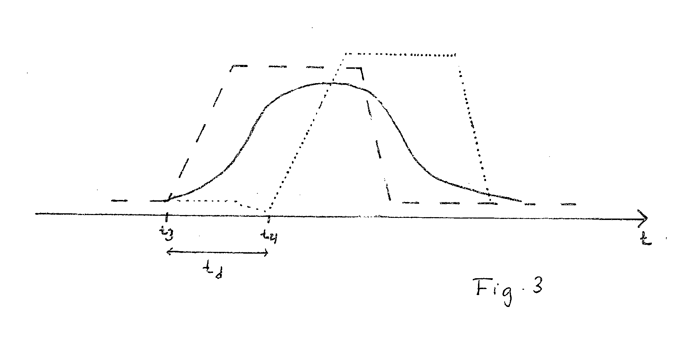

Figure 3, like Figure 2, illustrates a situation in which the pneumatic trigger function is based on pressure measurements. In Figure 3, the trigger function is not sensitive enough, causing the inspiration to be triggered too late in the breathing cycle compared to the patient's own breathing. Three curves are shown varying along a time axis denoted t. As in Figure 2, the solid curve represents the Edi signal recorded in the patient, that is, it reflects the patient's own breathing activity. The positive flank represents an inhalation by the patient. The dashed curve is an ideal ventilator cycle, starting inspiration (positive flank) when the patient starts inhaling. In Figure 3, the dotted curve is an example of a delayed triggering that will result if the trigger condition is too insensitive. In this case, when the patient starts inhaling, at a point in time t3, the pressure will drop but not enough to trigger an inspiration phase in the ventilator at once. Only at a second point in time t4 will the ventilator start its inspiration phase. Hence, there will be a time delay td between the point in time t3 when the patient starts to inhale and the point in time t4 when the ventilator starts an inspiration.

In both the cases illustrated in Figures 2 and 3, the correct triggering point in time, that is the point in time when the patient starts to inhale, can be determined by means of an Edi signal recorded on the patient. By monitoring the Edi signal, the point in time when the patient starts to inhale can be determined, as the start of the positive flank of the Edi signal shown in Figures 2 and 3.

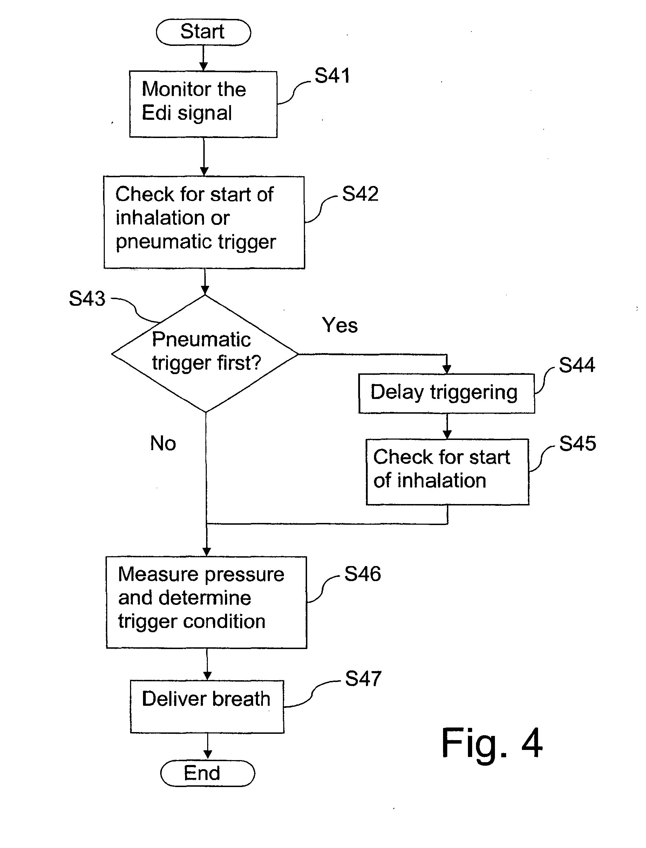

A first preferred embodiment of the inventive method is shown in figure 4. In this embodiment, as well as Figures 2 and 3 above, the triggering is based on pressure measurements. The skilled person can easily modify this to triggering on flow criteria instead, or on a combination of flow and pressure criteria, if the ventilator supports this.

To initiate the method, in step S41, the Edi signal is monitored during at least one breath in the patient. In step S42, either the point in time when the Edi signal indicates patient inhalation during this breath, is determined, that is, the point in time in which the Edi signal raised above a certain predetermined value, or the point in time when the pneumatic trigger condition is reached, whichever occurs first. In step S43, preferably, it is determined if the ventilator is triggered before the start of inhalation. If yes, the triggering has to be delayed, in step S44. The point in time when the Edi signal indicates patient inhalation is then determined in step S45. After step S45, or after step S43 if the triggering was not too early, the pressure in the ventilator at the starting time of inhalation is measured in step S46. This pressure, that is, the pressure at the actual start of inspiration by the patient, is used in step S46 as an indicator of what the pneumatic trigger condition should be. Finally, in step S47 the new trigger condition is set to be used in the following breaths, or presented to the operator as a proposed new setting.

The method may be performed during one breath only, or may be performed during several breaths to obtain an average measured value. Such an average value will probably provide a more correct value of the pressure in the ventilator at the onset of the patient's own inspiration than a measured value obtained during only one breath. In both cases, the method may be performed again at certain time intervals to ensure correct timing of the breathing support. Alternatively, the procedure may be performed again if the difference between the start of the breathing cycle of the ventilator and that of the patient becomes too big.

The adjustment procedure may be initiated by an operator. Instead of automatic adjustment, the operator can also use the result to adjust the trigger condition manually, thereby adjusting the timing of the breathing support cycle.

In step S44 the triggering of the ventilator should be delayed so as to enable correct measurement of the pressure and/or flow at the point in time when the patient starts to inhale. Therefore, the triggering should not be performed until after the patient's inhalation has started. However, a maximum delay should be set, to ensure that the breathing support delay will not be harmful to the patient.

The correction of the trigger condition, based on the value determined in step S47, may be carried out in different ways. The trigger condition, which, in the case of pressure triggering, will be a pressure value, which may be set as a function of the pressure measured in step S46.

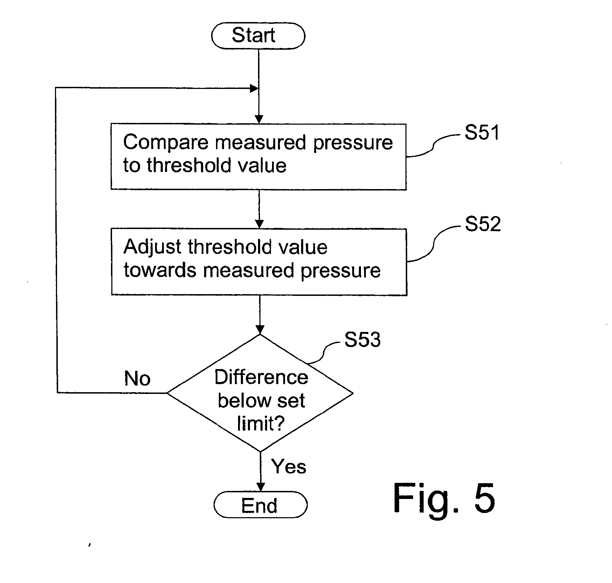

It may be favourable to adjust the trigger condition in several steps. In this case, step S47 will comprise the following substeps, illustrated in Figure 5:
In step S51 comparing the pressure measured in step S43 to the actual trigger condition currently applied in the ventilator.
In step S52 adjusting the trigger condition in the direction of the measured pressure value. If the measured pressure value is lower than the pressure value that will trigger the inspiration phase, the threshold value should be lowered. If the measured pressure value is higher than the threshold pressure that will trigger the inspiration phase, the threshold value should be raised. The change in the threshold value may, however, be carried out stepwise, so that the trigger conditions will be refined gradually. The steps could be carried out, for example, in fixed increments, for example, 0.1 cmH2O at a time, or as a fraction of the difference, for example 10% of the determined difference each time. The procedure may be iterated a predetermined number of times, or until the difference between the trigger conditions and the measured pressure is within an acceptable interval. This is indicated by decision step S53 in Figure 5, which terminates the procedure if the difference is below a set limit and returns to step S51 if the difference is still too large. Instead of determining the difference between actual pressure and threshold value, in the decision step S53 the difference in time between the start of the patient's own inhalation and the start of the inspiration phase of the ventilator could be evaluated, that is, the difference between the first and second points in time t1 and t2, or the difference between the third and fourth points in time t3 and t4, as the case may be. In this case, if the time difference is longer than a predetermined time, for example 100 ms, the procedure of Figure 5 should be reiterated. The predetermined time could be of the order of magnitude of 100 ms. It may be determined as a fix value, or based on duration of the patient's own breathing cycle, or inspiration phase.

Preferably, a pressure and/or flow interval is defined in which the trigger condition can be set, to avoid setting the trigger condition to a value that may be harmful to the patient.

Also, in step S44 a maximum delay should be set for the pneumatic triggering to avoid losing an entire breath. This maximum delay could be, for example 300 ms. It could also be based on a measured duration of the patient's breathing cycle or inspiration phase. If no Edi triggering has occurred after the maximum delay, then the triggering value could be set to the value measured in the ventilator at the maximum delay and this could be used as an initial value. If no breathing activity can be detected from the Edi signal, a breath should still be delivered to the patient within a suitable time.

A minimum pressure should be set, which will always trigger the ventilator, even when the maximum delay has not been exceeded. This should correspond to the least sensitive pressure that is allowed.
In order to evaluate the result of the adjustment, the time difference between the pneumatic control of the ventilator and the Edi signal may be determined continuously or at certain time intervals. In this way the changes in the time difference over time can be monitored and appropriate action can be taken when needed.

The Edi signal is prone to disturbances, for example, from stronger bioelectric signals in the patient's body. To avoid using an erroneous Edi signal as a basis for the trigger conditions, an automatic adjustment should not be allowed if the time difference between inspiration phase triggered by the ventilator and the patient's own inhalation is too great. Alternatively a quality indicator for the Edi signal could be used, to ensure that the Edi signal actually reflects the patient's breathing activity, and not an artefact.

Before starting the actual adjustment of the trigger conditions, by performing the steps of Figure 4, it may be useful to measure the Edi signal and the ventilator's breathing cycle for some breaths to compare the timing of the two. This comparison will indicate if the inspiration support is triggered too early or too late compared to the patient's own breathing activity, thereby indicating in which direction the trigger condition should be adjusted. Such a comparison can also be performed continuously, or at certain time intervals, to evaluate the need for adjusting the trigger conditions. If the timing of the Edi signal and the breathing support cycle differs less than a certain limit no adjustment is needed. If the difference in timing exceeds this limit an adjustment procedure as the one shown in Figure 4 should be performed.

The difference between the inspired and expired volumes may be used to evaluate whether there is any leakage before the trigger condition is adjusted. If a considerable leak is present the sensitivity of the adjusted trigger should preferably be limited.

As mentioned above, the triggering may be based on pressure or flow of gas in the ventilator. In the case of a leakage, pressure triggering will be more suitable than flow triggering, since a leakage will cause a flow, even if there is no patient activity.

## Claims

1. A computer program product comprising a computer readable medium having stored thereon computer readable code means for controlling a ventilator providing breathing support in a pneumatically triggered support mode to a patient, said computer program product comprising computer readable instructions which, when run in a control unit controlling a ventilator will cause the control unit to perform the following steps:
- obtaining a first measurement value of a bioelectric signal representative of the patient's own breathing function;
- determining, based on the bioelectric signal, at least one point in time in which the patient starts inhalation,
- obtaining a second measurement value related to a pressure and/or flow in the ventilator, to be used for determining a threshold value for triggering an inspiration phase in the ventilator, during said at least one point in time,
- determining the threshold value, to be used as trigger condition for the inspiration phase, on the basis of the second measurement value,
- using the trigger condition for initiating inspiration when ventilating the patient in support mode.

2. A computer program product according to claim 1, wherein the code means comprises computer readable instructions which, when run in a control unit controlling a ventilator will cause the control unit to determine the trigger condition on the basis of several measurement values, each obtained at a point in time when the patient starts inhalation, in different breaths.

3. A computer program product according to claim 1 or 2, wherein the code means comprises computer readable instructions which, when run in a control unit controlling a ventilator will cause the control unit to adjust the trigger condition by an amount determined on the basis of the measurement value.

4. A computer program product according to any one of the preceding claims, wherein the code means comprises computer readable instructions which, when run in a control unit controlling a ventilator will cause the control unit, after determining the start of inhalation and before measuring the pressure and/or flow, to determine whether an inspiration phase in the ventilator was triggered before the start of inhalation and, if so, slightly delaying the ventilator's inspiration phase until the patient's own breathing attempt can be detected.

5. A computer program product according to any one of the preceding claims, wherein the code means comprises computer readable instructions which, when run in a control unit controlling a ventilator will cause the control unit to adjust the trigger condition incrementally in such a way as to reduce the time difference between start of the ventilator's inspiration phase and the start of the patient's inspiration.

6. A computer program product according to claim 5, arranged to adjust the trigger condition in fixed increments.

7. A computer program product according to claim 5, arranged to adjust the trigger condition in increments determined on the basis of the difference between the measurement value and the trigger condition.

8. A control unit for a ventilator for use in support mode, said control unit comprising a computer program product according to any one of the claims 1-7, and processing means for controlling the ventilator in accordance with the instructions of the computer program product.

9. A ventilator for providing breathing assistance in support mode to a patient, said ventilator comprising a control unit according to claim 8 for controlling the ventilator.

## Patentansprüche

1. Computerprogrammprodukt mit einem computerlesbaren Medium, das darauf gespeichert computerlesbare Codemittel zum Steuern eines Beatmungsgeräts aufweist, das einem Patienten in einem pneumatisch ausgelösten Unterstützungsmodus eine Atmungsunterstützung bereitstellt, wobei das Computerprogrammprodukt computerlesbare Anweisungen umfasst, die dann, wenn sie in einer Steuereinheit laufen, die ein Beatmungsgerät steuert, die Steuereinheit veranlassen, die folgenden Schritte auszuführen:
- Beschaffen eines ersten Messwertes eines bioelektrischen Signals, das kennzeichnend für die eigene Atmungsfunktion des Patienten ist,
- Bestimmen mindestens eines Zeitpunkts, in dem der Patient das Einatmen beginnt, auf Basis des bioelektrischen Signals;
- Beschaffen eines zweiten Messwertes, der einen Druck und/oder einen Durchfluss in dem Beatmungsgerät betrifft, um zur Bestimmung eines Schwellenwertes für das Auslösen einer Ansaugphase im Beatmungsgerät während mindestens eines Zeitpunkts verwendet zu werden,
- Bestimmen des Schwellenwertes, der als eine Auslösebedingung für die Ansaugphase zu verwenden ist, auf Basis des zweiten Messwertes,
- Verwenden der Auslösebedingung für den Start des Ansaugens, wenn der Patient im Unterstützungsmodus beatmet wird.

2. Computerprogrammprodukt nach Anspruch 1, wobei die Codemittel computerlesbare Anweisungen aufweisen, die beim Ablauf in einer Steuereinheit, die ein Beatmungsgerät steuert, die Steuereinheit veranlassen, die Auslösebedingung auf Basis mehrerer Messwerte zu bestimmen, von denen jeder in verschiedenen Atemzügen zu einem Zeitpunkt bestimmt wurde, wenn der Patient das Einatmen beginnt.

3. Computerprogrammprodukt nach Anspruch 1 oder 2, wobei die Codemittel computerlesbare Anweisungen aufweisen, die beim Ablauf in einer Steuereinheit, die ein Beatmungsgerät steuert, die Steuereinheit veranlassen, die Auslösebedingung um einen Betrag anzupassen, der auf Basis des Messwertes bestimmt wurde.

4. Computerprogrammprodukt nach einem der vorherigen Ansprüche, wobei die Codemittel computerlesbare Anweisungen aufweisen, die beim Ablauf in einer Steuereinheit, die ein Beatmungsgerät steuert, die Steuereinheit veranlassen, nach dem Bestimmen des Beginns des Einatmens und vor dem Messen des Drucks und/oder Durchflusses zu bestimmen, ob eine Ansaugphase im Beatmungsgerät vor dem Beginn des Einatmens ausgelöst wurde, und wenn das so ist, die Ansaugphase des Beatmungsgeräts zu verzögern, bis der eigene Atmungsversuch des Patienten erfasst werden kann.

5. Computerprogrammprodukt nach einem der vorherigen Ansprüche, wobei die Codemittel computerlesbare Anweisungen aufweisen, die beim Ablauf in einer Steuereinheit, die ein Beatmungsgerät steuert, die Steuereinheit veranlassen, die Auslösebedingung schrittweise derart anzupassen, dass die Zeitdifferenz zwischen dem Beginn der Ansaugphase des Beatmungsgeräts und dem Beginn des Einatmens des Patienten verringert wird.

6. Computerprogrammprodukt nach Anspruch 5, das eingerichtet ist, die Auslösebedingung in festgelegten Schrittgrößen anzupassen.

7. Computerprogrammprodukt nach Anspruch 5, das eingerichtet ist, die Auslösebedingung in Schrittgrößen anzupassen, die auf Basis der Differenz zwischen dem Messwert und der Auslösebedingung bestimmt wurden.

8. Steuereinheit für ein Beatmungsgerät zur Verwendung in einem Unterstützungsmodus, wobei die Steuereinheit ein Computerprogrammprodukt nach einem der Ansprüche 1-7 und Verarbeitungsmittel aufweist, um das Beatmungsgerät gemäß den Anweisungen des Computerprogrammprodukts zu steuern.

9. Beatmungsgerät zur Bereitstellung einer Atemhilfe in einem Unterstützungsmodus für einen Patienten, wobei das Beatmungsgerät eine Steuereinheit nach Anspruch 8 aufweist, um das Beatmungsgerät zu steuern.

## Revendications

1. Produit programme informatique comprenant un support qui peut être lu par un ordinateur, dans lequel sont stockés des moyens de code qui peuvent être lus par un ordinateur, destiné à commander un insufflateur qui fournit à un patient une assistance respiratoire dans un mode assistance au patient déclenché de manière pneumatique, ledit produit programme informatique comprenant des instructions qui peuvent être lues par un ordinateur et qui, quand elles sont exécutées dans une unité de commande qui commande un insufflateur, font exécuter par l'unité de commande les étapes suivantes consistant à :
- obtenir une première valeur de mesure d'un signal bioélectrique représentatif de la fonction respiratoire propre au patient ;
- déterminer, sur la base du signal bioélectrique, au moins un instant où le patient débute une inhalation ;
- obtenir une seconde valeur de mesure qui se rapporte à une pression et / ou à un flux dans l'insufflateur, destinée à être utilisée de façon à déterminer une valeur de seuil pour le déclenchement d'une phase d'inspiration dans l'insufflateur, au cours du au moins un instant ;
- déterminer la valeur de seuil, à utiliser en tant que condition de déclenchement de la phase d'inspiration, sur la base de la seconde valeur de mesure ;
- utiliser la condition de déclenchement pour lancer une inspiration lors de la ventilation du patient dans le mode assistance.

2. Produit programme informatique selon la revendication 1, dans lequel les moyens de code comprennent des instructions qui peuvent être lues par un ordinateur et qui, quand elles sont exécutées dans une unité de commande qui commande un insufflateur, font déterminer par l'unité de commande la condition de déclenchement sur la base de plusieurs valeurs de mesure, chacune d'elles étant obtenue à un instant où le patient débute une inhalation, au cours de différentes respirations.

3. Produit programme informatique selon la revendication 1 ou la revendication 2, dans lequel les moyens de code comprennent des instructions qui peuvent être lues par un ordinateur et qui, quand elles sont exécutées dans une unité de commande qui commande un insufflateur, font régler par l'unité de commande la condition de déclenchement d'une quantité déterminée sur la base de la valeur de mesure.

4. Produit programme informatique selon l'une quelconque des revendications précédentes, dans lequel les moyens de code comprennent des instructions qui peuvent être lues par un ordinateur et qui, quand elles sont exécutées dans une unité de commande qui commande un insufflateur, font déterminer par l'unité de commande, après avoir déterminé le début de l'inhalation et avant la mesure de la pression et / ou du flux, si une phase d'inspiration dans l'insufflateur a été déclenchée avant le début de l'inhalation et, si c'est le cas, font retarder légèrement la phase d'inspiration de l'insufflateur jusqu'à ce que la tentative de respiration propre au patient puisse être détectée.

5. Produit programme informatique selon l'une quelconque des revendications précédentes, dans lequel les moyens de code comprennent des instructions qui peuvent être lues par un ordinateur et qui, quand elles sont exécutées dans une unité de commande qui commande un insufflateur, font régler par l'unité de commande la condition de déclenchement de manière incrémentale de façon à réduire la différence de temps entre le début de la phase d'inspiration de l'insufflateur et le début de l'inspiration du patient.

6. Produit programme informatique selon la revendication 5, agencé de façon à régler la condition de déclenchement selon des incréments fixes.

7. Produit programme informatique selon la revendication 5, agencé de façon à régler la condition de déclenchement selon des incréments déterminés sur la base de la différence entre la valeur de mesure et la condition de déclenchement.

8. Unité de commande d'un insufflateur destiné à être utilisé dans un mode assistance, ladite unité de commande comprenant un produit programme informatique selon l'une quelconque des revendications 1 à 7, et des moyens de traitement destinés à commander l'insufflateur selon les instructions du produit programme informatique.

9. Insufflateur destiné à fournir à un patient une assistance respiratoire dans un mode assistance, ledit insufflateur comprenant une unité de commande selon la revendication 8 destinée à commander l'insufflateur.
